# EUROPEAN PATENT APPLICATION

(11) **EP 1 949 844 A1**
(43) Date of publication of application: **30.07.2008**
(21) Application number: 05805950.2
(22) Date of filing: 14.11.2005
(51) Int. Cl.: A61B 1/00

(54) **METHOD OF ENDOSCOPICAL DIAGNOSIS OR TREATMENT AND MEDICAL DEVICE**

(71) Applicant: OLYMPUS MEDICAL SYSTEMS CORP., Shibuya-ku Tokyo 151-0072 (JP)
(72) Inventor: GOTO, Hiroaki, Hachioji-shi, Tokyo 193-0943 (JP); YUASA, Masaru, Hachioji-shi, Tokyo 192-0032 (JP); NISHINA, Kenichi, Hachioji-shi, Tokyo 192-0914 (JP); SAITO, Tatsuya, Setagaya-ku, Tokyo 156-0055 (JP)
(74) Representative: von Hellfeld, Axel
(86) International application number: PCT/JP2005/020868
(87) International publication number: WO 2007/055032

(57) **Abstract**

A method of performing diagnosis or treatment perendoscopically in which a guide catheter is inserted inside a body, and the guide catheter is made to protrude beyond a distal end portion of the endoscope; and in which a first diagnosis or treatment is performed by inserting a medical treatment device that performs the diagnosis or treatment of the body inside the guide catheter, and causing this medical treatment device to protrude by a first predetermined distance from the guide catheter; and in which the medical treatment device is extracted from the guide catheter; and in which a second diagnosis or treatment is performed by inserting a medical treatment device into the guide catheter, and causing the medical treatment device to protrude by a second distance which is adjusted in accordance with the first predetermined distance.

## Description

### TECHNICAL FIELD

The present invention relates to a method of performing diagnosis or treatment perendoscopically, and to a medical treatment device that is used when a body is being diagnosed or treated.

### BACKGROUND ART

When diagnosis or treatment of a peripheral bronchial tube of a bronchial tube is being performed, treatment is often performed by inserting an endoscope through a natural orifice of a body. At this time, it is necessary to insert a medical treatment device through a treatment channel of the endoscope, and guide the medical treatment device to a desired position of the complexly branching peripheral bronchial tube. Accordingly, firstly, an inductor is inserted under X-ray radioscopy as far as the vicinity of a lesion portion. The inductor has a bendable working portion provided at a distal end thereof. Once the working portion of the inductor has reached a branching point of the peripheral bronchial tube, the working portion is bent towards the peripheral bronchial tube which is the object of the diagnosis or treatment, and the inductor is subsequently pushed forward (see, for example, Patent document 1). After this, the inductor is used as a guide and the guide catheter is inserted as far as the vicinity of the object member. The inductor is then extracted from the endoscope. When, for example, tissue of a lesion portion is to be sampled, biopsy forceps are inserted through the guide catheter as far as the vicinity of the object portion.
[Patent document 1] Japanese Patent Application, First Publication No. 2004-154485

### DISCLOSURE OF INVENTION

### Problems to be Solved by the Invention

However, in the process to perform a diagnosis or treatment, when the inductor and the other medical treatment devices used for the diagnosis or treatment are being switched, it is difficult to keep the working portion at the distal end of each medical treatment device constantly in a desired position, and a skilled hand is required. Each time a medical device is inserted, it is necessary to confirm the position of the working portion by X-ray radioscopy, and each time the medical device is inserted into a biological subject, the position thereof needs to be adjusted. Because of this, workability is poor.
The present invention was conceived in consideration of the above described circumstances and it is an object thereof to enable position adjustment of the distal end working portion to be carried out easily and accurately when the medical treatment device is inserted or removed a plurality of times in the biological subject.

### Means for Solving the Problem

The present invention is a method of performing diagnosis or treatment perendoscopically that includes: a step in which a guide catheter is inserted inside a body following an endoscope that has been inserted into the body, and the guide catheter is made to protrude beyond a distal end portion of the endoscope; a step in which a first diagnosis or treatment is performed by inserting a medical treatment device that performs the diagnosis or treatment of the body inside the guide catheter, and causing this medical treatment device to protrude by a first predetermined distance from the guide catheter; a step in which the medical treatment device is extracted from the guide catheter; and a step in which a second diagnosis or treatment is performed by inserting a medical treatment device into the guide catheter, and causing the medical treatment device to protrude by a second distance which is adjusted in accordance with the first predetermined distance.

When diagnosis or treatment is performed a plurality of times, a medical treatment device is used in which the amount that it protrudes from a guide catheter is adjusted in advance. Even when the medical treatment device is switched, because each medical treatment device protrudes from the guide catheter by a distance that corresponds to that medical treatment device, the diagnosis or treatment is performed quickly and reliably. Here, the first predetermined distance and the second predetermined distance may be the same distance. In addition, the number of times that diagnosis or treatment is performed by means of a medical treatment device is not limited to two times.

In the present invention, it is also possible for the step in which the first diagnosis or treatment is performed and the step in which the second diagnosis or treatment is performed to be performed using different medical treatment devices. In this case, the diagnosis or treatment which is performed on the first occasion is different from that performed on the second occasion.

The present invention is a medical treatment device that includes: an insertion portion that is capable of being inserted into an internal hole in a guide catheter that is inserted into a body, and at whose distal end is provided a working portion that performs diagnosis or treatment of the body; and a position adjustment component that is capable of moving from a position where it has been fitted onto an outer circumference of the guide catheter to a position where an amount that the insertion portion is inserted relative to the guide catheter is a predetermined amount.

When diagnosis or treatment is performed using this medical treatment device, as a result of the medical treatment device protruding by a predetermined distance from the guide catheter, the position adjustment component is slid and the guide catheter and the medical treatment device are engaged together via the position adjustment component.

The present invention is a medical treatment device that includes: an insertion portion that is capable of being inserted into an internal hole in a guide catheter that is inserted into a body, and at whose distal end is provided a working portion that performs diagnosis or treatment of the body; and a position adjustment component that is fitted onto the insertion portion so as to be capable of moving in the longitudinal direction of the insertion portion, and is fixed to the insertion portion at a position determined by an operator before the insertion portion is inserted into the guide catheter, and is formed so as to be able to press against the guide catheter.

When diagnosis or treatment is performed using this medical treatment device, the guide catheter is inserted after the position of the position adjustment component has been adjusted in advance to a position suitable for the diagnosis or treatment. When the position adjustment component comes up against the guide catheter, the distal end portion of the medical treatment device protrudes by a predetermined length from the guide catheter. Because the position of the position adjustment component does not change even when the medical treatment device is extracted from the guide catheter, even if a medical treatment device is once again inserted into the guide catheter, there is no change in the amount of protrusion of the distal end portion of the medical treatment device.

### Effects of the Invention

According to the present invention, when a plurality of diagnoses or treatments are made of a lesion portion, because the distal end portion of a medical treatment device protrudes from a guide catheter by a length that has been adjusted in advance, positioning of the medical treatment device can be reliably carried out. Because the positioning adjustment that needs to be made each time a medical treatment device is replaced can be carried out easily, the time required for an operation can be reduced. Moreover, the effects from the x-ray radiation can be reduced.

### BRIEF DESCRIPTION OF DRAWINGS

[FIG. 1] FIG. 1 is a diagram showing the structure of an endoscope that is used in an embodiment of the present invention.
[FIG. 2] FIG. 2 is a diagram showing the structure of a curette which is an example of an inductor serving as a medical treatment device.
[FIG. 3] FIG. 3 is a diagram showing a guide catheter.
[FIG. 4] FIG. 4 is a diagram showing the structure of a medical treatment device system when the medical treatment device is an ultrasonic probe.
[FIG. 5] FIG. 5 is a diagram showing the structure of a medical treatment device system when the medical treatment device is a biopsy forceps.
[FIG. 6] FIG. 6 is a diagram showing the structure of a medical treatment device system when the medical treatment device is a cytological diagnosis brush.
[FIG. 7] FIG. 7 is a diagram showing in typical form a bronchial tube serving as an example of a treatment object.
[FIG. 8] FIG. 8 is a diagram showing an endoscope inserted in a bronchial tube with the distal end portion thereof in contact with and fixed in the bronchial tube.
[FIG. 9] FIG. 9 is a diagram showing a curette inserted as far as a lesion portion.
[FIG. 10] FIG. 10 is a diagram showing a curette used as a guide with the guide catheter inserted as far as a lesion portion.
[FIG. 11] FIG. 11 is a diagram showing a curette being extracted while the guide catheter is left in place.
[FIG. 12] FIG. 12 is a diagram showing an ultrasonic probe inserted through a guide catheter to a lesion portion.
[FIG. 13] FIG. 13 is a diagram showing a placement of a position adjustment component in the case of FIG. 12.
[FIG. 14] FIG. 14 is a diagram when a distal end position of a guide catheter is accurately set.
[FIG. 15] FIG. 15 is a diagram showing a biopsy forceps inserted through a guide catheter to a lesion portion.
[FIG. 16] FIG. 16 is a diagram used to illustrate a task when an amount of protrusion of an operating portion is adjusted by the position adjustment component.
[FIG. 17] FIG. 17 is a diagram showing the position adjustment component being slid towards a base end side.
[FIG. 18] FIG. 18 is a diagram showing the position adjustment component which has been slid to the base end side being pressed against the guide catheter causing the operating portion to protrude considerably.
[FIG. 19] FIG. 19 is a diagram showing the endoscope in the bronchial tube fixed in position by a balloon provided in the endoscope.
[FIG. 20] FIG. 20 is a diagram illustrating the task of fixing the endoscope using an anchoring component that is provided in an endoscope insertion portion.
[FIG. 21] FIG. 21 is a diagram showing an endoscope fixed that is fixed by an anchoring component and by the friction force of a mouthpiece.
[FIG. 22] FIG. 22 is a diagram showing a guide catheter fixed to the endoscope by a clip.
[FIG. 23] FIG. 23 is a diagram illustrating another embodiment of the position adjustment component.
[FIG. 24] FIG. 24 is a diagram showing state in which a guide catheter and a medical treatment device are anchored via a position adjustment component.
[FIG. 25] FIG. 25 is a diagram showing a position adjustment component being slid from a guide catheter side to a medical treatment device side.
[FIG. 26] FIG. 26 is a diagram illustrating another embodiment of the position adjustment component.
[FIG. 27] FIG. 27 is a cross-sectional view of FIG. 26.

### DESCRIPTION OF THE REFERENCE NUMERALS

- 1: Endoscope
- 10: Curette (Medical treatment device)
- 12, 33: Insertion portion
- 17,35: Catheter
- 20: Guide catheter
- 30, 50, 60: Medical treatment device system
- 31: Ultrasonic probe (Medical treatment device)
- 40, 82, 90: Position adjustment component
- 51: Biopsy forceps (Medical treatment device)
- 61: Cytological diagnosis brush (Medical treatment device)

### BEST MODE FOR CARRYING OUT THE INVENTION

Embodiments of the present invention will now be described in detail with reference made to the drawings.

### (First embodiment)

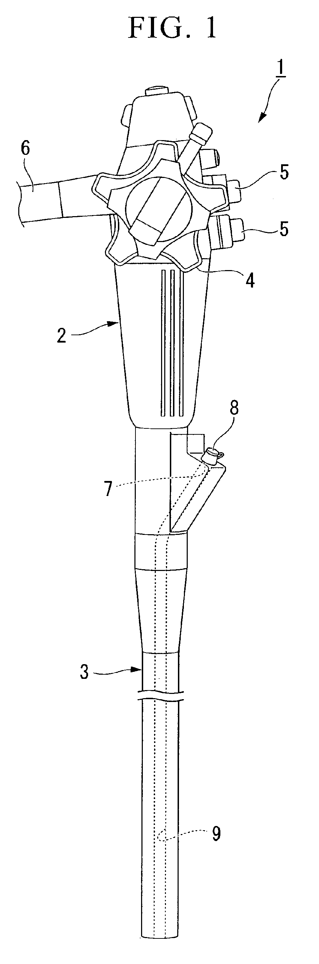

An endoscope that is used in the present embodiment is shown in FIG. 1. An endoscope 1 has an endoscope operating unit 2 that is operated by an operator, and a flexible endoscope insertion portion 3 that is inserted into a body extends from the endoscope operating unit 2. An angle knob 4 that bends a distal end portion of the endoscope portion 3, a switch 5 and the like are provided on the endoscope operating unit 2. In addition, a control unit, a display unit, and the like (not shown) are connected by a universal cable 6. An insertion aperture 7 is formed in a side portion of the endoscope operating unit 2. The insertion aperture 7 is an aperture on the extracorporeal side of a working channel 9. A forceps plug 8 is fitted into the insertion aperture 7. The working channel 9 passes through the interior of the endoscope insertion portion 3 and opens at the distal end thereof. Medical treatment devices, guide catheters (described below) and the like are inserted through this working channel 9.

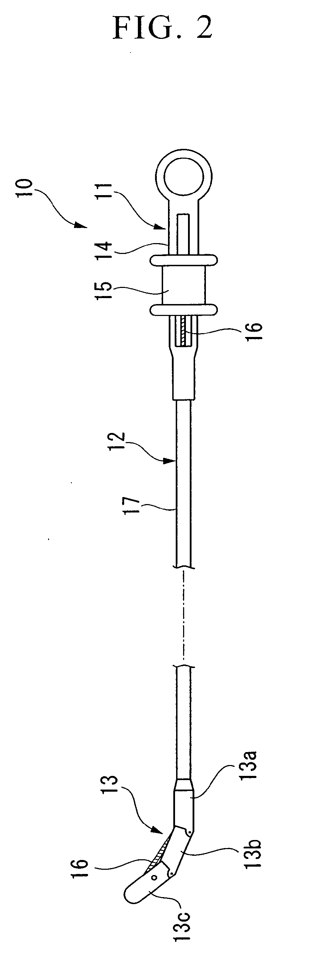

A curette which is a medical treatment device used for cell abrasion and has a cup and a bending mechanism at its distal end is an example of a medical treatment device.
As is shown in FIG. 2, in a curette 10, an elongated, flexible insertion portion 12 extends from the operating unit 11 which is operated by the operator. A working portion 13 is provided at the distal end of the insertion portion 12. The operating unit 11 has an operating unit main body 14, and a slider 15 is mounted on the operating unit main body 14 such that it can move forward and rearward directions. A ring for manipulation is provided on a base end side of the operating unit main body 14. The slider 15 is able to move forward and rearward directions in the longitudinal direction of the operating unit main body 14, namely, in the longitudinal direction of the insertion portion 12. An end portion of an operating wire 16 is fixed to the slider 15. The operating wire 16 is inserted into a catheter 17 of the insertion portion 12 such that it can move forward and rearward directions.

The insertion portion 12 has a structure that enables the operating wire 16 to be inserted through the catheter 17. The catheter 17 is manufactured from a flexible, elongated material. A distal end portion of the operating wire 16 is drawn into the working portion 13. The working portion 13 has a structure in which three arm portions 13a, 13b, and 13c are joined together by pins in this order from the insertion portion 12 side. The arm portion 13a on the distal end side is formed as a cup that is used when abraded tissue is being sampled. The operating wire 16 passes through the interior of these arm portions 13a, 13b, and 13c. The operating wire 16 is fixed to the arm portion 13c that is located on the furthest distal end side. Accordingly, when the operating wire 16 is pulled, the working portion 13 is able to bend around the fulcrums formed by the respective pins. Furthermore, when the operating wire 16 is restored, the bent working portion 13 can be extended substantially in a straight line. Note that the working portion 13 may also be formed by a single body that has deformable thin portions.

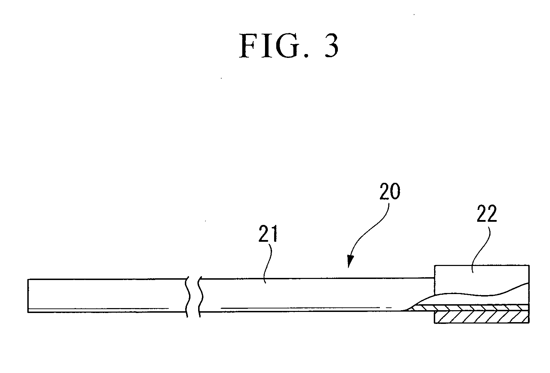

This curette 10 is used by being inserted through a guide catheter 20 such as that shown in FIG 3. The guide catheter 20 has a flexible tube 21, and a base end portion 22 thereof has an outer circumference whose diameter has been enlarged. The inner diameter of the tube 21 is large enough to allow the insertion portion 12 of the curette 10 to be inserted therein such that it can move forward and rearward directions. The outer diameter of the tube 21 is less than the diameter of the working channel 9. The outer diameter of the base end portion 22 is larger than the diameter of the working channel 9. The length of the guide catheter 20 is longer than that of the working channel 9. Note that the base end portion 22 is fixed to the tube 21, however, it may also be formed as a single unit with the tube 21.

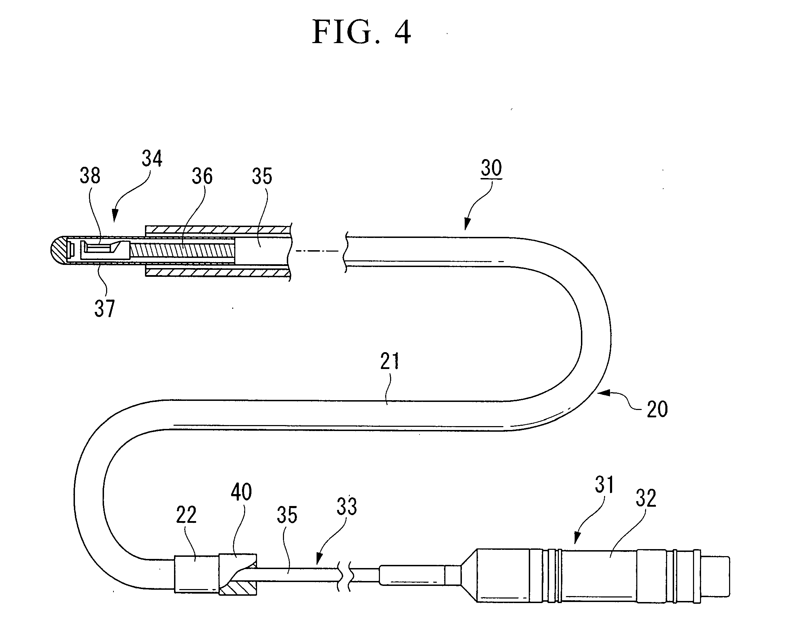

Various medical treatment device systems are shown in FIG. 4 through FIG. 6.
A medical treatment device 30 shown in FIG. 4 has a guide catheter 20, and an ultrasonic probe 31 that is used by being inserted in the guide catheter 20. The ultrasonic probe 31 is a medical treatment device that has a connector portion 32, an elongated, flexible insertion portion 33 that extends from the connector portion 32, and a working portion 34 that is provided at the distal end of the insertion portion 33. The connector portion 33 is mechanically and electrically connected to a drive apparatus (not shown).
In the insertion portion 33, a flexible shaft 36 is rotatively inserted through the interior of the catheter 35. The flexible shaft 36 is inserted inside a cover 37 of the working portion 34. An ultrasonic transducer 38 is mounted onto a distal end portion of the flexible shaft 36 inside the cover 37. The ultrasonic transducer 38 is an element that emits ultrasonic waves after receiving signal transmissions from a cable (not shown) that passes through the interior of the flexible shaft 36, and receives ultrasonic waves that have returned after striking an object and converts these ultrasonic waves into electrical signals. The interior of the cover 37 is filled with an ultrasonic wave medium such that ultrasonic waves can be easily transmitted.

The catheter 35 of the insertion portion 33 has an outer diameter that enables it to be inserted into an internal hole in the guide catheter 20. A position adjustment component 40 is slidably fitted onto an outer circumference of a base end portion side of the catheter 35. The position adjustment component 40 is a ring-shaped component having a hole through which the catheter 35 passes, and the outer diameter thereof is larger than that of the base end portion 22 of the guide catheter 20. The position adjustment component 40 is manufactured from a resin which is able to expand and contract. This position adjustment component 40 is able to be moved manually by the operator in the longitudinal direction of the catheter 35. The position adjustment component 40 remains in position when the operator stops manually moving the position adjustment component 40. The position where the position adjustment component 40 is installed is adjusted to a position where, when the ultrasonic probe 31 has been inserted through the guide catheter 20, the working portion 34 protrudes from the distal end portion of the guide catheter 20 by a first predetermined distance. The position adjustment component 40 does not move during the process in which the ultrasonic probe 31 is inserted in the guide catheter 20, or in the process in which it is removed from the guide catheter 20, and has sufficient engaging force with the catheter 35 to allow it to remain in its adjusted position.

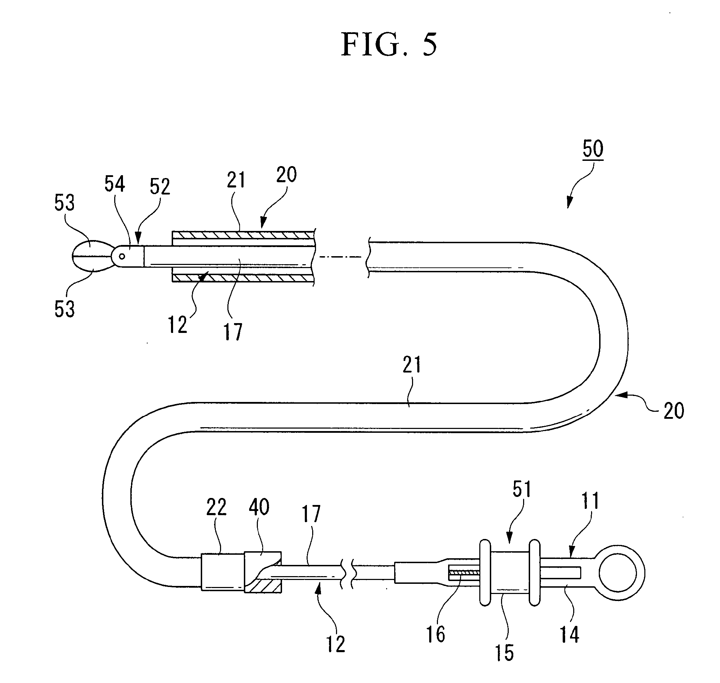

A medical treatment device 50 shown in FIG. 5 has a guide catheter 20, and a biopsy forceps 51 that is used by being inserted into the guide catheter 20. The biopsy forceps 51 is a medical treatment device that has an operating unit 11, an elongated, flexible insertion portion 12 that extends from the operating unit 11, and a treatment portion (i.e., a working portion) 52 that is provided at the distal end of the insertion portion 12. The insertion portion 12 is constructed with the operating wire 16 passing through the interior of the catheter 17. The operating wire 16 joins together the slider 15 of the operating unit 11 and a pair of biopsy cups 53 of the treatment portion 52. In the treatment portion 52, the pair of biopsy cups 53 are mounted on a supporting component 54 such that they can open and close freely. Each biopsy cup 53 is joined to the operating wire 16 via a linking mechanism (not shown). When the operating wire 53 is moved forward, the pair of biopsy cups 53 are opened, while when the operating wire 53 is moved backward, the pair of biopsy cups 53 are closed.

The catheter 17 of the insertion portion 12 has an outer diameter that enables it to be inserted into the internal hole in the guide catheter 20. A position adjustment component 40 is slidably fitted onto an outer circumference of a base end portion side of the catheter 17. The position where the position adjustment component 40 is installed is adjusted to a position where, when the biopsy forceps 51 has been inserted through the guide catheter 20, the treatment portion 52 protrudes from the distal end portion of the guide catheter 20 by a second predetermined distance. The second distance may be the same as or may be different from the first distance.

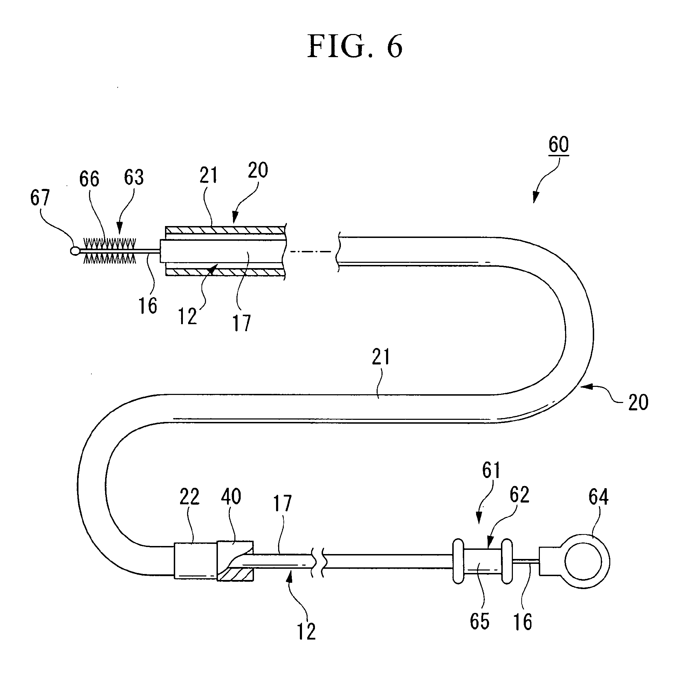

A medical treatment device 60 shown in FIG. 6 has a guide catheter 20, and a cytological diagnosis brush 61 that is used by being inserted into the guide catheter 20. The cytological diagnosis brush 61 is a medical treatment device that has an operating unit 62, an elongated, flexible insertion portion 12 that extends from the operating unit 62, and a treatment portion (i.e., a working portion) 63 that is provided at the distal end portion of the insertion portion. In the insertion portion 12, the operating wire 16 passes through the interior of the catheter 17. The operating wire 16 penetrates an operating unit main body 65 of the operating unit 62 such that it can move forward and rearward directions, and a manipulation ring 64 is fixed to an end portion of the operating wire 16 that extends from the operating unit main body 65. A brush 66 is attached to a distal end portion of the operating wire 16. The brush 66 is able to be housed inside the catheter 17 of the insertion portion 12. When the brush 66 is housed inside the insertion portion 12, a distal end tip 67 thereof strikes against the distal end of the insertion portion 12.

The catheter 17 of the insertion portion 12 has an outer diameter that enables it to be inserted into the internal hole in the guide catheter 20. A position adjustment component 40 is fitted onto an outer circumference of a base end portion side of the catheter 17. The position where the position adjustment component 40 is installed is adjusted to a position where, when the cytological diagnosis brush 61 has been inserted through the guide catheter 20, the distal end portion of the treatment portion 63 or the insertion portion 12 protrudes from the distal end portion of the guide catheter 20 by a third predetermined distance. The third distance may be the same as the first distance and the third distance or may be different therefrom.

Note that in the biopsy forceps 51 and the cytological diagnosis brush 61 as well, the position adjustment component 40 does not move during the process in which the biopsy forceps 51 and the cytological diagnosis brush 61 are inserted into the guide catheter 20, or in the process in which they are removed from the guide catheter 20, and has sufficient engaging force with the catheter 17 to allow it to remain in its adjusted position.

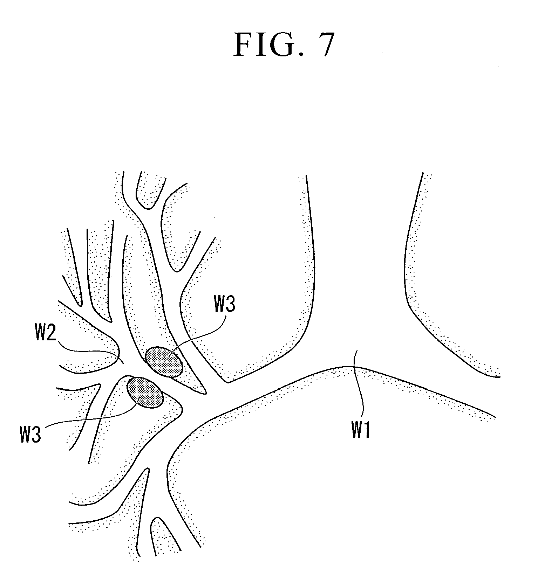

Next, an operation of the present embodiment will be described. Note that in the description given below, as is shown in FIG. 7, an example is described of a case in which diagnosis and treatment are performed on lesion portions W3 (i.e., subject portions) occurring in a peripheral bronchial tube W2 of a bronchial tube W1 of a human body.

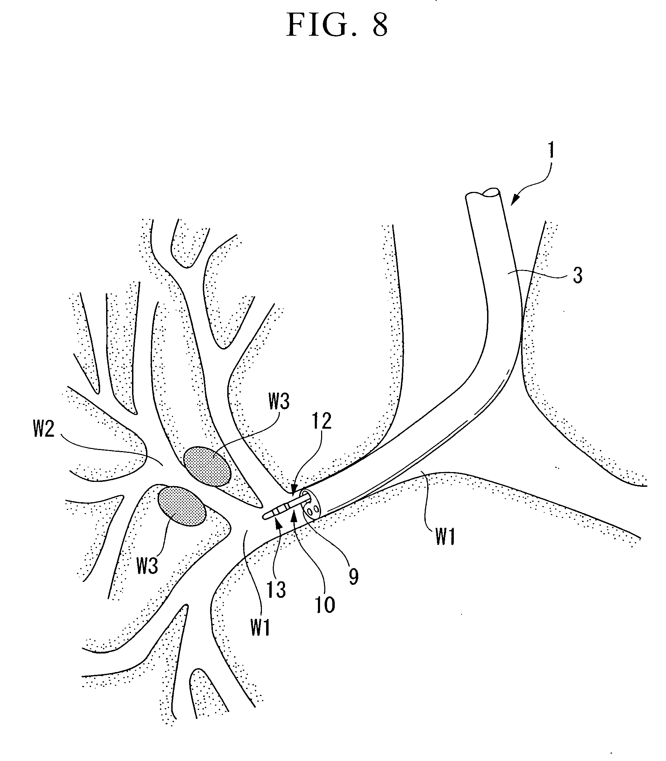

As is shown in FIG. 8, the endoscope 1 is inserted into a bronchial tube W1. The endoscope 1 is inserted through a natural orifice of a patient, namely, through either the mouth or nose. Because the bronchial tube W1 becomes narrower as it moves inwards, the insertion of the endoscope 1 is stopped when the distal end portion of the endoscope insertion portion 3 comes up against an inner wall of the bronchial tube W1. At this time, the endoscope insertion portion 3 is fixed by friction to the bronchial tube W1.

Once the endoscope 1 has been fixed in position relative to the bronchial tube W1, the operator inserts the curette 10 into the working channel 9. The curette 10 is used as an inductor to guide the guide catheter 22 the lesion portions W3. Accordingly, the insertion portion 12 is inserted in advance into the guide catheter 20 before the curette 10 is inserted into the endoscope 1. This causes the curette 10 to protrude from the distal end of the endoscope 1 while irradiating x-rays on to the bronchial tube W1 and verifying an image of the area around the lesion portions W3. At a branch portion W11 of the peripheral bronchial tube W2, the slider 15 of the operating unit 11 shown in FIG. 2 is pulled so that the operating wire 16 is drawn back towards the operator, thereby bending the working portion 13. After the distal end portion of the bent working portion 13 has been positioned so as to face towards the peripheral bronchial tube W2 where the lesion portions W3 are located, the entire curette 10 is moved forward. As a result, the curette 10 is inserted into the peripheral airway to W2 where the lesion portions W3 are located.

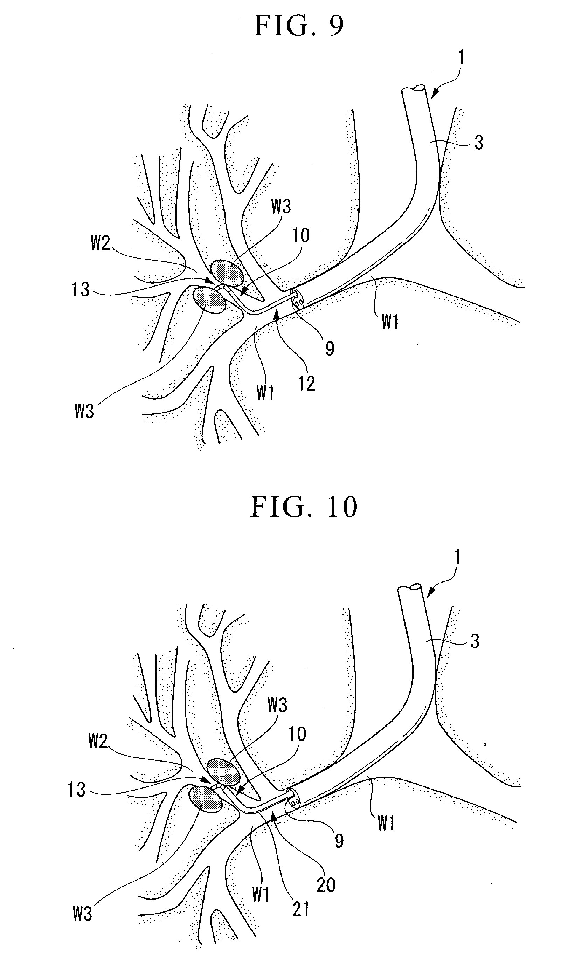

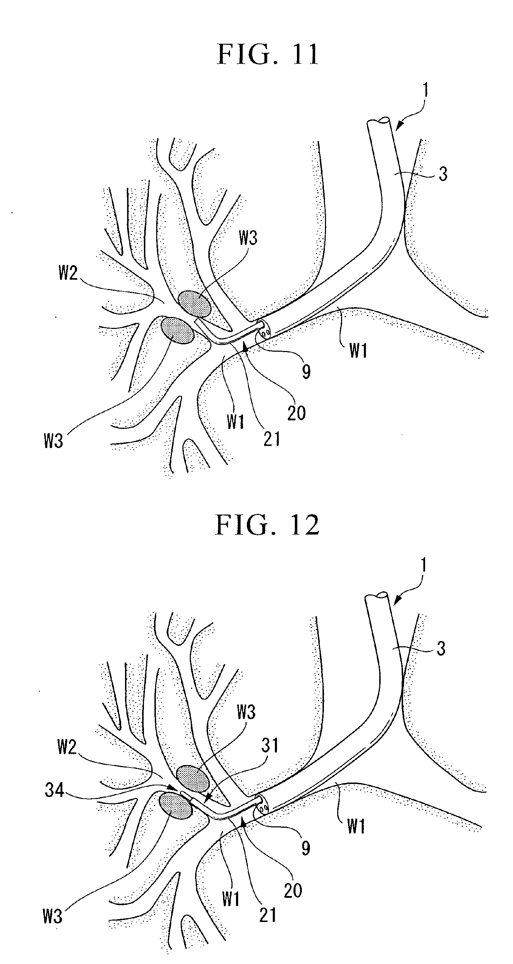

As is shown in FIG. 9, when the working portion 13 of the curette 10 reaches the lesion portions W3, the guide catheter 20 is pushed therein. Alternatively, if the guide catheter 20 is moved forward together with the curette 10, then it is not necessary to perform the pushing in operation. As is shown in FIG. 10, the guide catheter 20 is pushed in until the distal end portion thereof reaches the vicinity of the lesion portions W3. The position of the distal end of the guide catheter 20 is confirmed through observation under x-ray irradiation. Thereafter, as is shown in FIG. 11, the curette 10 is removed by itself from inside the body while the guide catheter 20 is left in position.

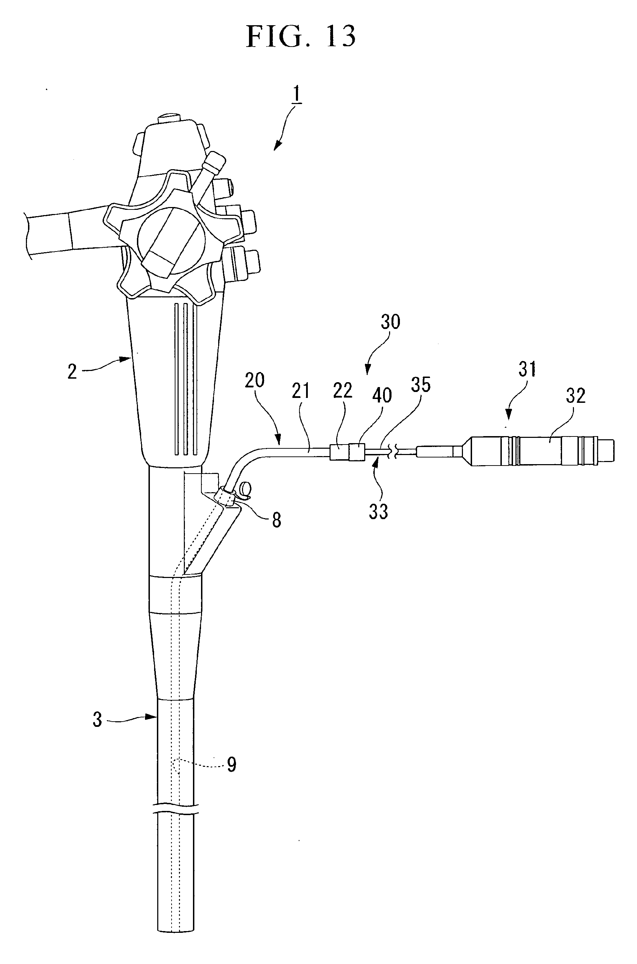

Next, as is shown in FIG. 12, the ultrasonic probe 31 is inserted through the guide catheter 20, and is inserted into the peripheral bronchial tube W2. The ultrasonic probe 31 is used as a medical treatment device that provides an image of the lesion portions W3. As is shown in FIG. 13, because the position adjustment component 40 is fitted in a pre-adjusted position on the insertion portion 33 of the ultrasonic probe 31, the ultrasonic probe 31 can be inserted until the position adjustment component 40 strikes against the base end portion 22 of the guide catheter 20. At this time, as is shown in FIG. 12, the working portion 34 at the distal end protrudes by the first distance from the guide catheter 20, and is placed in a position which corresponds substantially to the center in the longitudinal direction of the lesion portions W3. A drive apparatus is installed in the connector portion 32 of the ultrasonic probe 31, and ultrasonic waves are irradiated outwardly in a radial direction while the ultrasonic transducer 38 is being rotated around its axis by means of the flexible shaft 36. When ultrasonic waves reflected by the peripheral bronchial tube W2 are received by the ultrasonic transducer 38, an extraction image of the lesion portions W3 is obtained. By using the ultrasonic probe 31, it is possible to confirm the precise positions and state of the lesion portions W3. For example, by employing x-ray irradiation, information concerning the depth direction, and information concerning positional relationships between the lesion portions W3 and blood vessels which are difficult to obtain can be obtained.

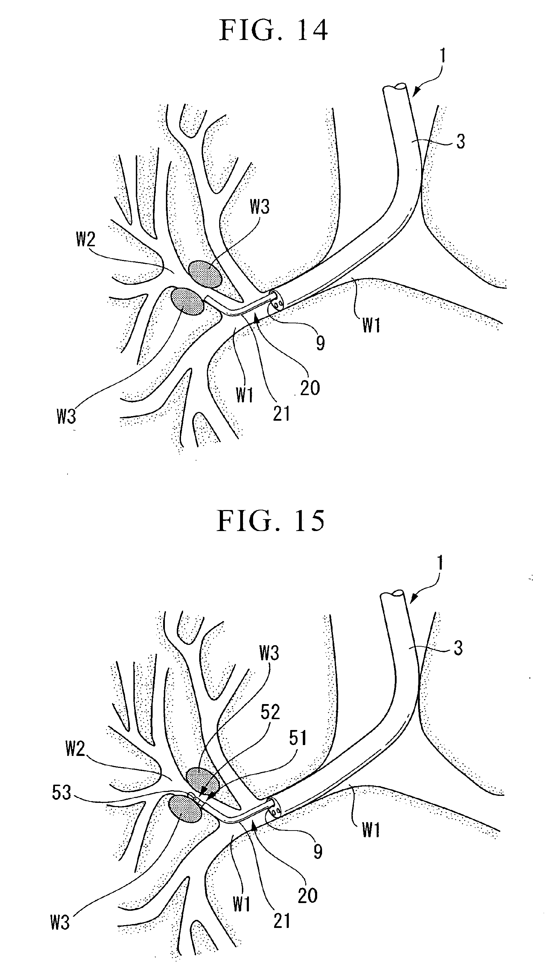

Positioning of the distal end of the guide catheter 20 is then carried out accurately while the operator confirms the provided image of the lesion portions W3. For example, the operator confirms the precise position of the lesion portions W3 by moving the ultrasonic probe 31 forwards and backwards in the longitudinal direction of the peripheral bronchial tube W2, and matches the position of the distal end portion of the guide catheter 20 to a position on the outer edge of the lesion portions W3. Once the positioning of the guide catheter 20 has ended, the guide catheter 20 is fixed relative to the endoscope 1. Friction fixing by means of the forceps plug 8 shown in FIG. 13 can be used in order to fix the guide catheter 20 in position. Once the guide catheter 20 is fixed, the ultrasonic probe 31 is withdrawn by itself. As is shown in FIG. 14, the guide catheter 20 is left behind in a state of being positioned in the vicinity of the lesion portions W3.

Thereafter, as is shown in FIG. 15, a medical treatment device in the form of the biopsy forceps 51 is inserted into the peripheral bronchial tube W2 through the guide catheter 20. Because the position adjustment component 40 is fitted onto a previously adjusted position on the insertion portion 12 of the biopsy forceps 51, if the biopsy forceps 51 is inserted until the position adjustment component 40 strikes against the base end portion 22 of the guide catheter 20, then the distal end treatment portion 52 protrudes by the pre-adjusted second distance from the guide catheter 20. This second distance is a position suitable for sampling the lesion portions W3, and a biopsy is performed at this position. If the slider 15 of the operating unit 11 is then moved forward, the operating wire 16 is pushed in and the pair of biopsy cups 53 are opened. If the slider is then pulled back after the biopsy cups 53 have been pressed against a lesion portion W3, the pair of biopsy cups 53 are closed around the tissue of the lesion portion W3. If the entire biopsy forceps 51 is then pulled back, the tissue inside the biopsy cups 53 is torn away. If the biopsy forceps 51 is then extracted from the endoscope 1, the tissue of the lesion portion W3 can be sampled. Note that because the guide catheter 20 is fixed relative to the endoscope 1, there is no change in the insertion position of the guide catheter 20.

When the tissues of the lesion portions W3 are to be sampled a plurality of times, the biopsy forceps 51 is again inserted into the endoscope 1. If the operator has not consciously moved the position adjustment component 40 and places the position adjustment component 40 up against the guide catheter 20, the biopsy is performed in substantially the same position. If a plurality of biopsies are to be performed at different positions in the longitudinal direction of the peripheral bronchial tube W2, a fine adjustment is made by moving the position adjustment component 40 manually in the longitudinal direction of the insertion portion. By moving the position adjustment component 40 backwards towards the base end side, a biopsy can be performed on tissue at a deeper position (i.e., a position further away from the endoscope 1). If the position adjustment component 40 is pushed out to the distal end side, then a biopsy can be performed on tissue at a shallower position (i.e., a position closer to the endoscope 1). If a plurality of biopsies are to be performed, then it is possible to either use the same biopsy forceps 51, or use different biopsy forceps 51 in sequence.

When the cytological diagnosis brush 61 shown in FIG. 6 is to be used, the cytological diagnosis brush 61 is inserted until the pre-adjusted position adjustment component 40 comes up against the base end portion 22 of the guide catheter 20. The distal end treatment portion 63 protrudes by the pre-adjusted third distance from the distal end portion of the guide catheter 20. This third distance is a position suitable for sampling the lesion portions W3, and a biopsy is performed at this position. If the manipulation ring 64 of the operating unit 62 is moved forwards relative to the operating unit main body 64, then the brush 66 protrudes from the insertion portion 12. After the brush 66 has scraped a lesion portion W3 and tissue has been sampled, then the cytological diagnosis brush 61 is extracted by itself from the endoscope 1 and the guide catheter 20 is left behind.

When cells are to be sampled a plurality of times using the cytological diagnosis brush 61, in the same way as in the case of the biopsy forceps 51, the cytological diagnosis brush 61 is inserted in or removed from the guide catheter 20. As long as the position of the position adjustment component 40 is not moved intentionally, the insertion position of the cytological diagnosis brush 61 remains constant.

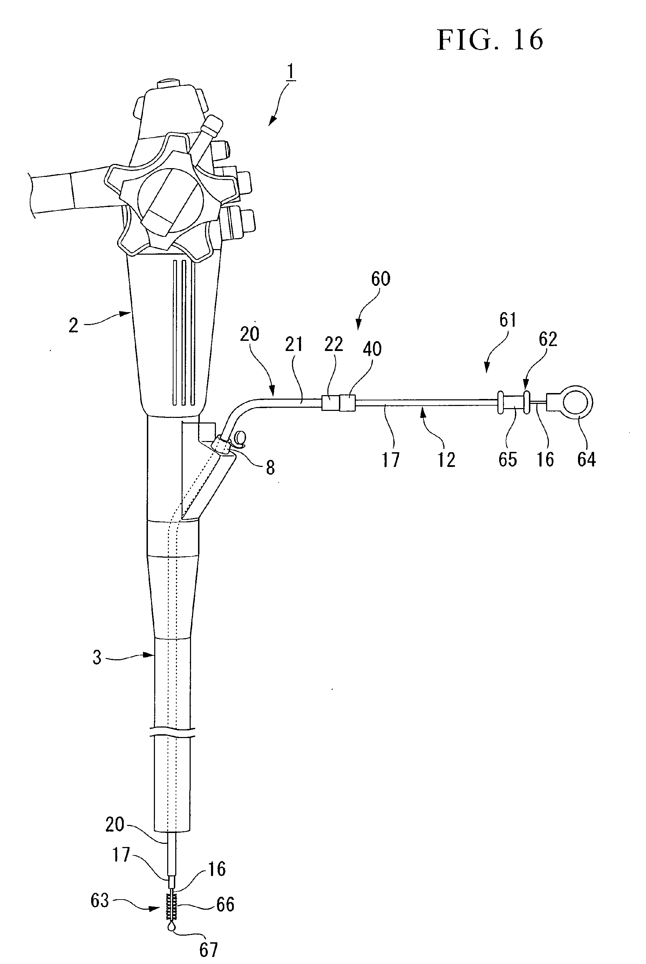

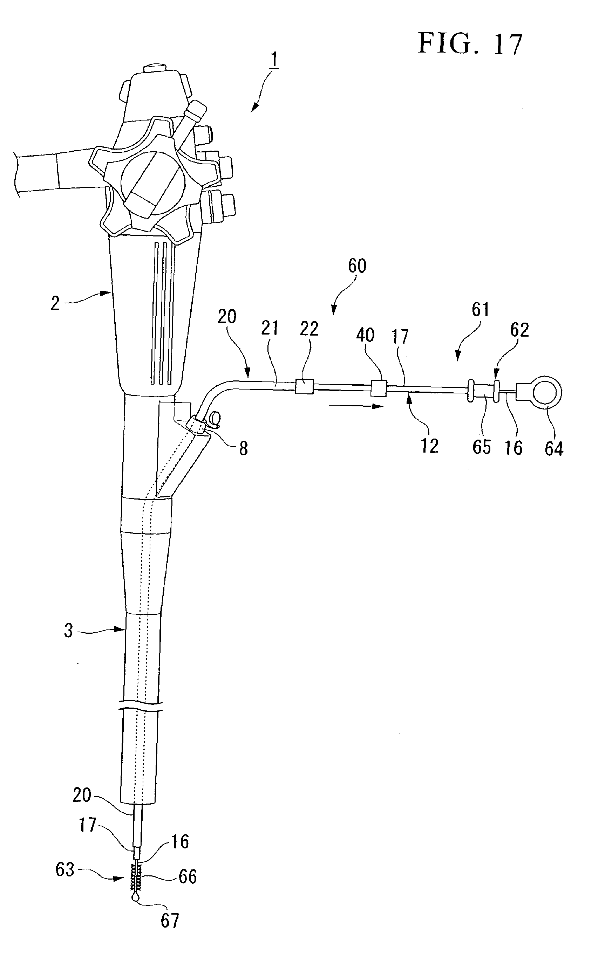

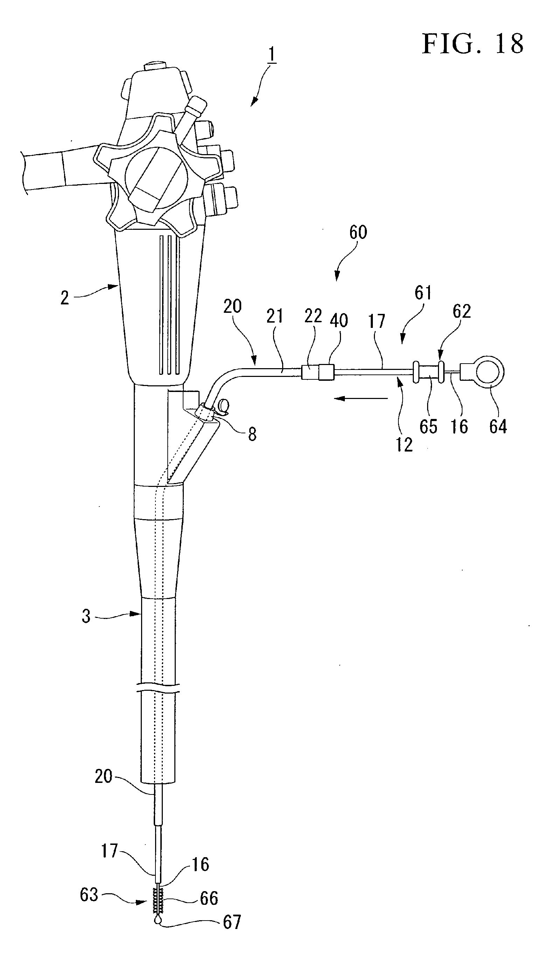

Here, the procedure when the position of the position adjustment component 40 is adjusted will be described with reference made to FIG. 16 through FIG. 18. Firstly, as is shown in FIG. 16, when the position adjustment component 40 comes up against the guide catheter 20, the distal end portion of the cytological diagnosis brush 61, which includes the treatment portion 63, protrudes by a predetermined distance from the distal end portion of the guide catheter 20. If the operator desires to make the treatment portion 63 protrude even further, as is shown in FIG. 17, the position adjustment component 40 is made to slide to the base end side by itself while the positions of the insertion portion 12 and the guide catheter 20 remain fixed. Once the position adjustment component 40 has been slid for the desired distance, it is stopped. As is shown in FIG. 18, if the cytological diagnosis brush 61 is pushed into the guide catheter 20, then the position adjustment component 40 which has completed its slide movement can be inserted until it strikes against the base end portion 22 of the guide catheter 20. The treatment portion 63 at this time protrudes further by the same distance that the position adjustment component 40 has been moved. This operation can be performed in reverse if a reduction of the protrusion amount of the treatment portion 63 is required.

According to this embodiment, by causing the guide catheter 20 to protrude from the endoscope 1 and inserting it as far as the vicinity of a lesion portion W3, it is possible to reliably introduce a medical treatment device even when a diagnosis or treatment is to be performed in a narrow lumen such as the peripheral bronchial tube W2 in a portion where the endoscope 1 cannot reach. In particular, when diagnosis or treatment is to be performed in which a medical treatment device is inserted and extracted a number of times, the medical treatment device can be inserted quickly into the vicinity of a lesion portion W3.

When the position adjustment component 40 is fitted onto a medical treatment device, and the position adjustment component 40 is placed at a position that is suitable for this medical treatment device, then it is possible to place the working portion in a predetermined position simply by placing the position adjustment component 40 against the guide catheter 20. Because the position adjustment component 40 does not move when the medical treatment device is extracted, the time taken up by manual operations can be shortened. When different type of medical treatment devices are to be used, by adjusting the position adjustment component in advance to a position that is suitable for the medical treatment device, it is possible to shorten the time required for manual operations. This adjustment may be performed in advance, or alternatively, it is also possible when the curette 10 is being inserted to confirm the position of the curette 10 and, based on this, to then finely adjust the position adjustment component 40.

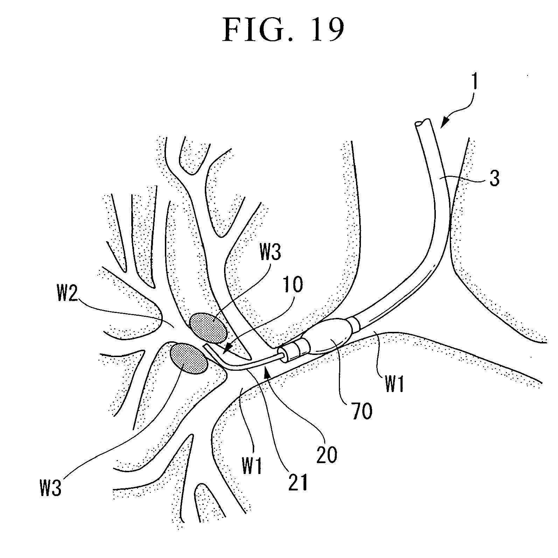

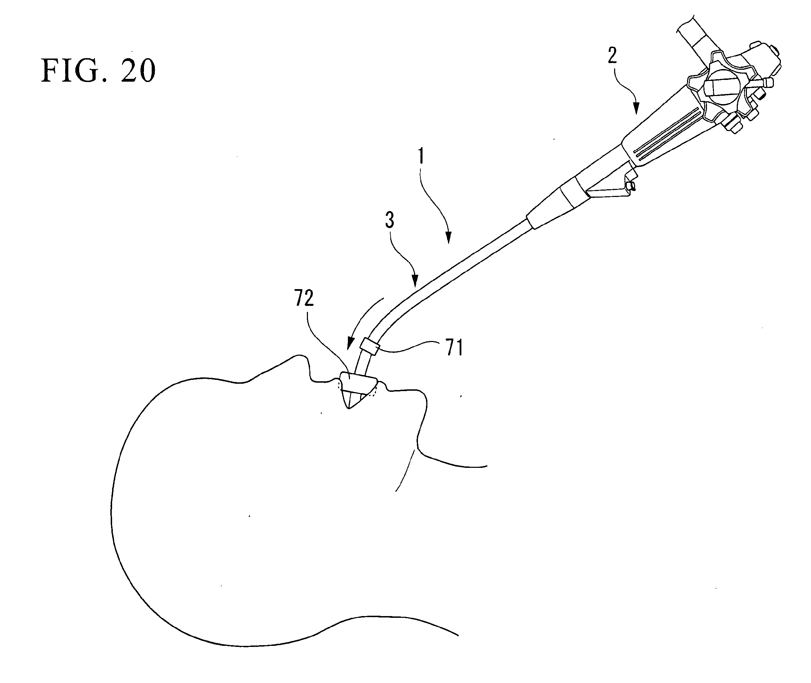

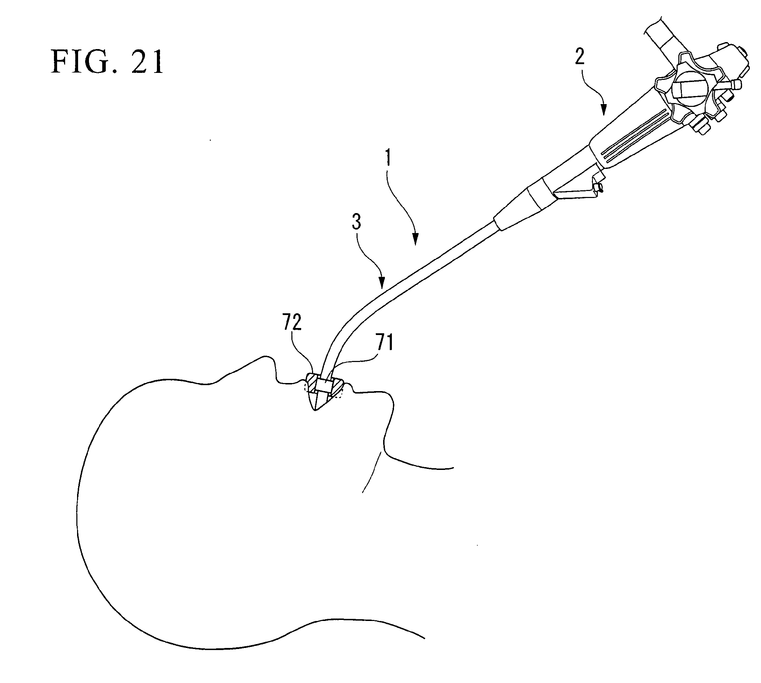

Here, a variant example of a method for fixing the endoscope 1 relative to the bronchial tube W1 is shown in FIG. 19 through FIG. 21. As is shown in FIG. 19, it is also possible to fit a balloon 70 onto an outer circumference of the distal end of the endoscope insertion portion 3. If fluid is supplied to the balloon 70 from outside the body through a catheter tube (not shown), the balloon 70 becomes inflated and touches the inner wall of the bronchial tube W1. As a result, the endoscope 1 is fixed by friction in the bronchial tube W1.
As is shown in FIG. 20, it is also possible to fit an endoscope engaging component 71 on the endoscope insertion portion 3. The endoscope engaging component 71 is formed by an elastic tube or the like, and is fixed by friction to an inner wall of a mouthpiece 72 which is fitted inside the patient's mouth.

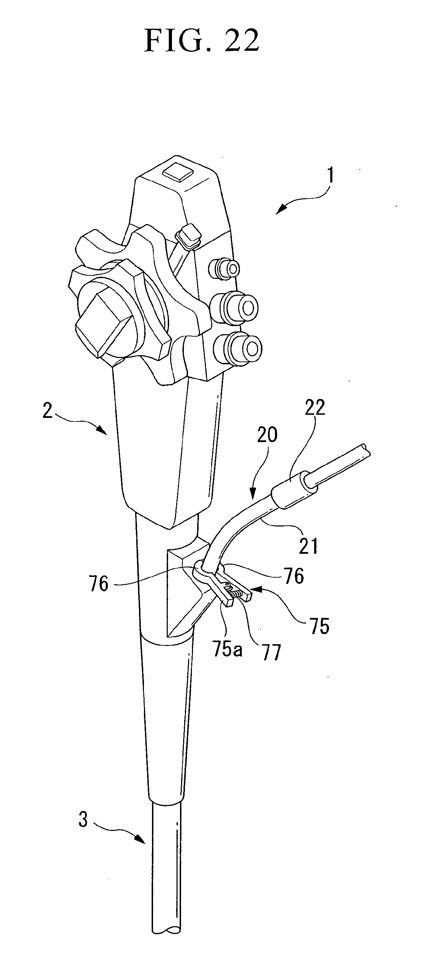

Moreover, the method used to fix the guide catheter 20 relative to the endoscope 1 is not limited to using the forceps plug 8, and it is also possible for it to be fixed by hand. Furthermore, a clip 75 such as that shown in FIG. 22 may also be used as a fixing component. Once the guide catheter 20 has been inserted a predetermined length into the working channel 9, an end portion 75a of the clip 75 is held so as to open up a gripping portion 76 which is then pressed against the boundary between the tube 21 of the guide catheter 20 and the forceps plug 8. If the operator then removes his hand from the end portion 75a of the clip 75, the clip 75 is closed by the urging force of the elastic component 77, and the guide catheter 20 and forceps plug 8 become gripped. As a result, the guide catheter 20 is positioned relative to the endoscope 1 and is fixed via the clip 75.

### (Second embodiment)

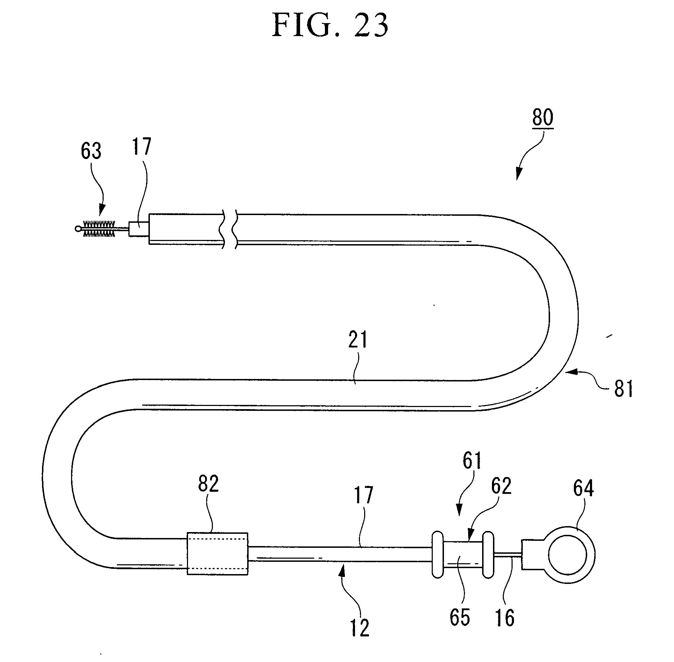

A second embodiment of the present invention will now be described with reference made to the drawings.
The structure of a medical treatment device system is shown in FIG. 23. A medical treatment device system 80 has a guide catheter 81, and the cytological diagnosis brush 61 which is used by being inserted in the guide catheter 81. The medical treatment device is not limited to the cytological diagnosis brush 61, and may also be the ultrasonic probe 31, or the biopsy forceps 51 or the like.
In the guide catheter 81, a position adjustment component 82 is fitted by press insertion or the like onto a base end portion of the tube 21. The position adjustment component 82 does not need to be fixed relative to the tube 21, and it is also possible for it to be able to slide in the longitudinal direction of the tube 21. Note that when it is removed from the tube 21, the inner diameter of the position adjustment component 82 is smaller than the outer diameter of the catheter 17 of the insertion portion 12 of the cytological diagnosis brush 61.

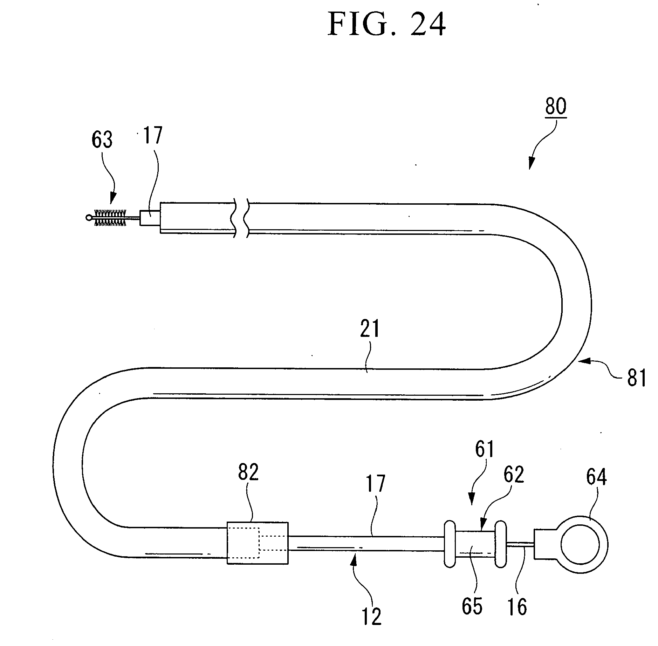

In this medical treatment device system 80, in the state shown in FIG. 23, the cytological diagnosis brush 61 is able to move forwards or backwards freely relative to the guide catheter 81. As is shown in FIG. 24, if the position adjustment component 82 is moved backwards so that a portion of the position adjustment component 82 is pushed out from the base end portion of the guide catheter 81, the portion that has been pushed out of the position adjustment component 82 is pressed against the outer circumference of the catheter 17 of the insertion portion 12 of the cytological diagnosis brush 61. As a result, the cytological diagnosis brush 61 is fixed on the guide catheter 81 via the position adjustment component 82. When the cytological diagnosis brush 61 is being inserted through the guide catheter 81, once the treatment portion 63 of the cytological diagnosis brush 61 is protruding from the guide catheter 81 by a predetermined amount needed for the treatment, the position adjustment component 82 is moved so that the cytological diagnosis brush 61 is fixed to the guide catheter 81. When the medical treatment device system 80 is being inserted into the working channel 9 of the endoscope 1, it is possible to guide the treatment portion 63 to a lesion portion W3 while the aforementioned predetermined amount is kept unchanged.

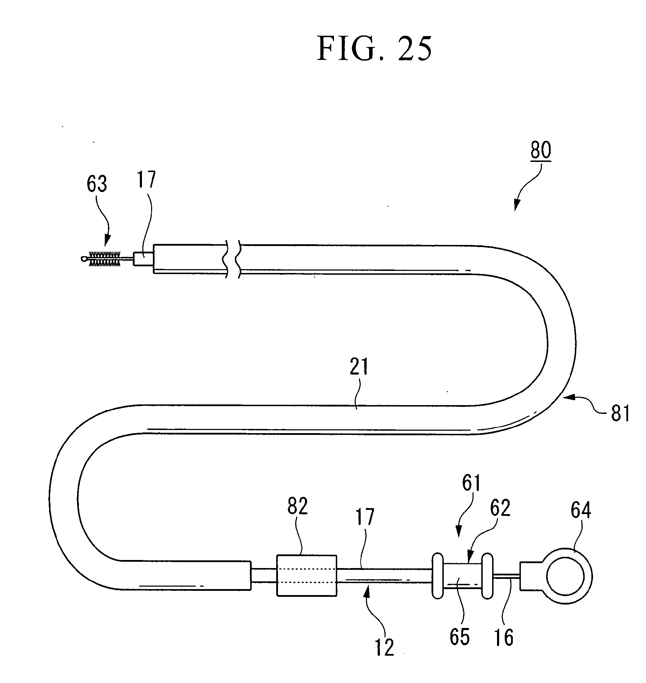

Moreover, as is shown in FIG. 25, it is also possible to move the entire position adjustment component 82 from the tube 82 of the guide catheter 81 completely onto the insertion portion 12 of the cytological diagnosis brush 61. The position adjustment component 82 is engaged at a pre-adjusted position. When the cytological diagnosis brush 61 is inserted into the guide catheter 81, and the position adjustment component 82 is placed against the base end portion of the guide catheter 81, the treatment portion 63 can be made to protrude by a predetermined distance from the distal end of the guide catheter 81.

### (Third embodiment)

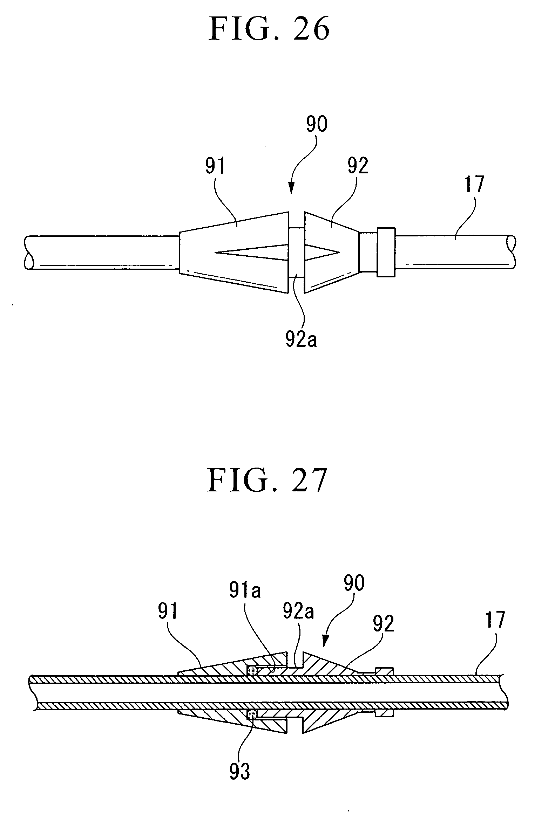

A position adjustment component according to the present embodiment is shown in FIG. 26 and FIG. 27. The position adjustment component 90 has a cylindrical outer component 91 and a cylindrical inner component 92 through which the catheter 17 of a medical treatment device is able to be inserted. A ring-shaped groove 91 a through which a distal end portion 92a of the inner component 92 is able to be inserted is formed in an inner circumference of a base end portion of the outer component 91. A receiving portion for an elastic ring 93 is formed in a distal end portion of this groove 91 a.
When the elastic ring 93 is housed in this receiving portion, an inner diameter thereof is substantially equivalent to the outer diameter of the catheter 17 when no external force is acting thereon. When the distal end portion of the inner component 92 is inserted into the groove 91 a of the outer component 91 and is pressed in tightly, the elastic ring 93 becomes squashed and presses against the catheter 17. As a result, the position adjustment component 90 becomes fixed in position on the catheter 17. If a structure is employed in which a state in which the distal end portion 92a of the inner component 92 and the groove 91 a of the outer component 91 are mutually engaged or fastened together by screws can be maintained, then a state in which the position adjustment component 90 is fixed in position on the catheter 17 can be maintained even when the operator removes his hand. By using this type of medical treatment device, the same operation and effects as in the first embodiment can be obtained.

Note that the present invention is not limited to the above described respective embodiments, and the present invention may be put to widespread use insofar as these do not depart from the spirit or scope of the present invention.
For example, the object of diagnosis or treatment is not limited to the bronchial tube W1, and maybe another lumen.
The medical treatment device system may also be constructed to include the curette 10 and catheter 20, and also the position adjustment component 40 or the position adjustment component 82. In addition, as the medical treatment device it is also possible to use a medical treatment laser probe or high-frequency forceps.
The positions where the position adjustment components 40 and 82 are used may be the positions to which they were adjusted during shipping, or may be adjusted by the operator.
Instead of being manufactured from cylindrical components, the position adjustment components may also be constructed so as to be fixed in position as a result of a predetermined position of the catheter of the insertion portion being gripped by a clip or the like.

### INDUSTRIAL APPLICABILITY

The present invention can be applied to devices that are inserted into a body to perform diagnosis or treatment, and to systems that include these devices.

## Claims

1. A method of performing diagnosis or treatment perendoscopically comprising:
a step in which a guide catheter is inserted inside a body following an endoscope that has been inserted into the body, and the guide catheter is made to protrude beyond a distal end portion of the endoscope;
a step in which a first diagnosis or treatment is performed by inserting a medical treatment device that performs the diagnosis or treatment of the body inside the guide catheter, and causing this medical treatment device to protrude by a first predetermined distance from the guide catheter;
a step in which the medical treatment device is extracted from the guide catheter; and
a step in which a second diagnosis or treatment is performed by inserting a medical treatment device into the guide catheter, and causing the medical treatment device to protrude by a second distance which is adjusted in accordance with the first predetermined distance.

2. The method of performing diagnosis or treatment perendoscopically according to claim 1, wherein the step in which the first diagnosis or treatment is performed and the step in which the second diagnosis or treatment is performed are performed using different medical treatment devices.

3. A medical treatment device comprising:
an insertion portion that is capable of being inserted into an internal hole in a guide catheter that is inserted into a body, and at whose distal end is provided a working portion that performs diagnosis or treatment of the body; and
a position adjustment component that is capable of moving from a position where it has been fitted onto an outer circumference of the guide catheter to a position where an amount that the insertion portion is inserted relative to the guide catheter is a predetermined amount.

4. A medical treatment device comprising:
an insertion portion that is capable of being inserted into an internal hole in a guide catheter that is inserted into a body, and at whose distal end is provided a working portion that performs diagnosis or treatment of the body; and
a position adjustment component that is fitted onto the insertion portion so as to be capable of moving in the longitudinal direction of the insertion portion, and is fixed to the insertion portion at a position determined by an operator before the insertion portion is inserted into the guide catheter, and is formed so as to be able to press against the guide catheter.
